# EUROPEAN PATENT APPLICATION

(11) **EP 1 705 175 A1**
(43) Date of publication of application: **27.09.2006**
(21) Application number: 05006577.0
(22) Date of filing: 24.03.2005
(51) Int. Cl.: C07C 271/22, C07C 229/22, C07C 69/38, C07D 207/34

(54) **Process for preparing C5 products and their use for Atorvastatin synthesis**

(71) Applicant: Ratiopharm GmbH, 89070 Ulm (DE)
(72) Inventor: Tararov, Vitali, Moscow (RU); Korostylev, Andrei, 390000 Ryazan (RU); Börner, Armin, 18059 Rostock (DE); Bobal, Pavel, 90101 Malacky (SK); Frantisek, Jaroslav, 61800 Brno (CZ); Stohandl, Jiri, Bobrova 236 (CZ); Denike, Kane, Georgetown, Ontario L7G5Y4 (CA); Jeker, Nicolas, 4143 Dornach (CH)
(74) Representative: Teipel, Stephan

(57) **Abstract**

The present invention relates to a process for preparing C5 intermediates and their use in the preparation of pyrrole derivatives of a class that is effective at inhibiting the biosynthesis of cholesterol in humans, and more particularly to improved synthetic methods for preparing 3,5-dihydroxy-7-pyrrol-1-yl heptanoic acids from 1,4-diketo starting materials. The invention further relates to intermediates in this process

## Description

The present invention relates to a process for preparing C5 intermediates useful for preparing pyrrole derivatives of a class that is effective at inhibiting the biosynthesis of cholesterol in humans, and more particularly to improved synthetic methods for preparing 3,5-dihydroxy-7-pyrrol-1-yl heptanoic acids from 1,4-diketo starting materials. The invention further relates to intermediates in this process.

It is known that certain 3,5-dihydroxy heptanoic acid derivatives are competitive inhibitors of the 3-hydroxy-3-methyl-glutaryl-coenzyme A ("HMG-CoA"). HMG-CoA is a key enzyme in the biosynthesis of cholesterol in humans. Its inhibition leads to a reduction in the rate of biosynthesis of cholesterol. The first HMG-CoA inhibitor to be described is compactin ([1 S-[1α(*R**), 7β, 8β(2*S**, 4*S**),8αβ]]-1,2,3,7,8a-hexahydro-7-methyl-8-[2-(tetrahydro-4-hydroxy-6-oxo-2*H*-pyran-2-yl)ethyl]-1-naphthalenyl 2-methylbutanoate), which was isolated from cultures of *Penicillium* in 1976. In 1987, lovastatin ([1S-[1α(*R**),3α, 7β, 8β(2*S**, 4*S**),8αβ]]-1,2,3,7,8,8a-hexahydro-3,7-dimethyl-8-[2-(tetrahydro-4-hydroxy-6-oxo-2*H*-pyran-2-yl)ethyl]-1-naphthalenyl 2-methylbutanoiate) became the first HMG-CoA reductase inhibitor approved by the Food and Drug Administration (FDA) for treatment of hypercholesterolemia. Both compactin and lovastatin are derived from bacterial cultures. Two other naturally-derived HMG-CoA reductase inhibitors, simvastatin and pravastatin are structurally related to compactin and lovastatin.

In 1987, it was reported in U.S. patent No. 4,681,893 that compounds within a certain class of 3,5-dihydroxy-7-pyrrol-1-yl heptanoic acid (and the corresponding lactones) also were effective at inhibiting the HMG-CoA reductase enzyme. One such compound is [*R*(*R**,*R**)]-2-(4-fluorophenyl)-β,δ-dihydroxy-5-(1-methylethyl)-3-phenyl-4-[(phenylamino)carbonyl]-1H-pyrrole-1-heptanoic acid ("atorvastatin"), which was said to provide surprising inhibition in U.S. patent No. 5,273,995. Atorvastatin later received FDA approval as an adjunct to a low cholesterol diet to reduce elevated levels of total cholesterol, low density lipoprotein cholesterol, apo B and triglycerides and to increase levels of high density lipoprotein cholesterol in patients with hyperlipidemia.

In contrast to compactin, lovastatin, simvastatin and pravastatin, there is no known fermentation culture that produces atorvastatin. It, and other 3,5-dihydroxy-7-pyrrol-1-yl heptanoic acids, must be synthesized by traditional synthetic methods.

A number of processes for the synthesis of 3,5-trihydroxy-7-pyrrol-1-yl heptanoic acids and in particular atorvastatin are known. Some of the processes are concerned with the synthesis of the 3,5-dihydroxy heptanoic acid side chain of the pyrrole ring while others are concerned with the formation of the pyrrole ring.

For example, EP-A-0 330 172 teaches that the pyrrole ring can be formed by the Paal-Knorr reaction between an 1,4-diketone and a primary amine being a precursor of the 3,5-dihydroxy heptanoic acid side chain. The 1,4-diketone already bears those substituents required at the pyrrole ring of atorvastatin and in particular the (phenylamino)carbonyl group required at position 4 of the pyrrole ring.

It is, however, difficult to obtain the required 1,4-diketone comprising the amino carbonyl moiety in good yield and purity. Moreover, even when starting from a commercially available 1,4-diketone precursor comprising the required (phenylamino)carbonyl side chain, it turned out to be difficult, if not impossible, to replace the phenylamino residue by other residues such as alkoxy or aryloxy residues without cleavage of the diketone. This limits the possible variation of the substituents and makes a screening for new compounds for inhibiting the biosynthesis of cholesterol in humans difficult.

Moreover, several approaches have been published to synthesize the C7 side chain on the 1 position of the pyrrole ring in atorvastatin. For example EP-A-0 409 281 suggests to first prepare the required 2,3,4,5-substituted pyrrole derivative bearing a C3 substituent on position 1. This C3 substituent is then converted in a C5 substituent and subsequently into the desired C7 substituent. This has, however, the disadvantage that the chain extension has to be carried out on the pyrrole derivative with its various other substituents making the reaction difficult to handle and bearing the risk of a potential partial loss of the valuable pyrrole derivative.

Therefore, there is still a need for further methods of synthesizing pyrrole derivatives and in particular intermediates for the preparation of 3,5-dihydroxy-7-pyrrole-1-yl heptanoic acids having HMG-CoA inhibitory activity.

It has now been found that several of the prior art problems can surprisingly be overcome by certain C5 intermediates, the reaction of these C5 intermediates with certain 1,4-diketones to pyrroles having a C5 substituent on position 1 and subsequent chain extension of the C5 substituents to the desired C7 substituent. In particular it has been found that the required C5 intermediates can be synthesized in preparative scale starting from β-alanine or Boc-β-AlaOH according to the following reaction scheme 1.

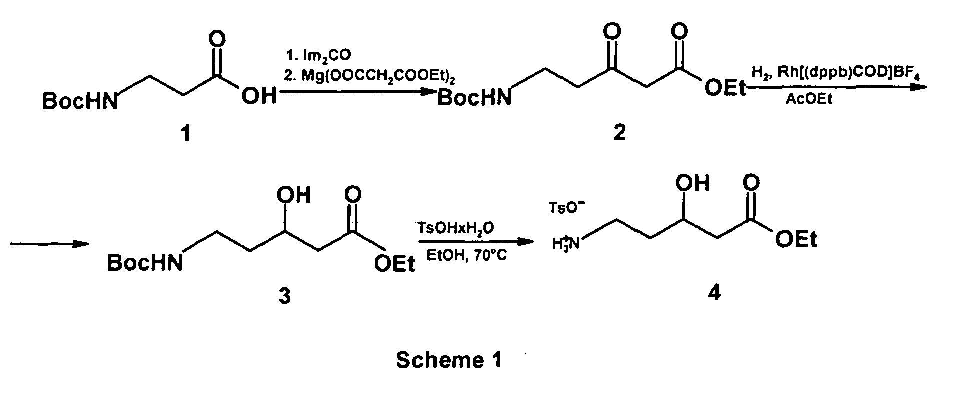

The advantage of this sequence is avoiding of additional purification of intermediate products. All reactions can be carried out at ambient conditions. The intermediate and final compounds have high purity (NMR).

Therefore, the present invention relates to a process for the preparation of a compound of the formula I 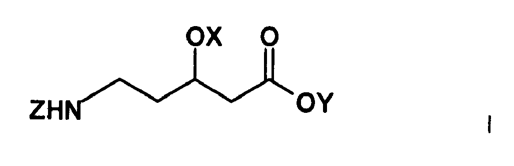 or a salt thereof, wherein Z is H or an amino protecting group, X is H or an hydroxy protection group, and Y is H or a carboxy protecting group, which process comprises the steps of
a) reacting a compound of the formula II 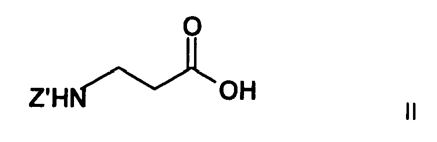 wherein Z' is an amino protecting group, with N,N'-carbonyldiimidazole (Im₂CO) and a compound of the formula III 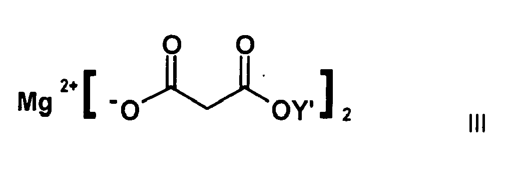 wherein Y' is a carboxy protecting group, to a compound of the formula IV 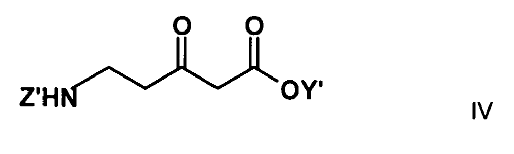 wherein Z' and Y' are defined as above,
b) reducing the compound of the formula IV to obtain a compound of the formula V 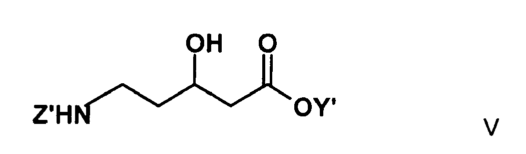 wherein Z' and Y' are defined as above, and
c) optionally deprotecting the amino group and/or the carboxy group of the compound of the formula V, optionally protecting the hydroxy group of the compound of the formula V and/or optionally converting the resulting compound into a salt thereof.

As protecting groups for the optionally protected hydroxy groups, the optionally protected carboxy groups and the optionally protected amino groups usual protecting groups known to the person skilled in the art may be used. Suitable protecting groups are exemplified in WO 03/044011, the content of which in incorporated by reference herein.

A preferred protecting group for Z and Z' is tert-butoxycarbonyl (Boc). Preferred protecting groups for Y and Y' are alkyl, aryl and aralkyl, such as straight, branched or cyclic C₁₋₁₀ alkyl, preferably C₁₋₆ alkyl, more preferably methyl, ethyl, iso-propyl or tert-butyl. Aryl can be for example phenyl or napththyl. Aralkyl can be for example aryl such as phenyl or naphthyl linked via a C₁₋₁₀ alkylen, preferably C₁₋₆ alkylen, such as benzyl.

The reduction step (b) in the process of the present invention, which preferably is a hydrogenation step, can be carried out under chiral or achiral conditions. Under achiral conditions a mixture of the two enantiomers of the compound of formula I is obtained. If it is desired to obtain the compound in its chiral form comprising mainly only one of its enantiomers, the reduction step (b) can be carried out under chiral conditions, for example in the presence of a chiral catalyst. Preferably the hydrogenation is carried out under homogenous achiral or chiral enantioselective conditions.

Suitable catalysts may be selected from iridium, rhodium and ruthenium complexes, such as Rh[(dppb)COD]BF₄ (wherein dppb = 1,4-bis(diphenylphosphino)butane and COD = 1,5-cyclooctadiene).

Preferably the ligand in the Rh, Rn and Ir complexes should be chiral. For example the following ligands can be used for Rh, Ru and Ir catalyzed asymmetric hydrogenation:
(*R*)-NORPHOS - (2*R*,3*R*)-(-)-2,3-bis(diphenylphosphanyl)-bicyclo[2.2.1]hept-5-ene.
(*R*,*R*)-CHIRAPHOS = (2*R*,3*R*)-(-)Bis(diphenylphosphanyl)butane
(*R*,*R*)-DEGUPHOS = (3*R*,4*R*)-(+)-1-Benzyl-3,4-bis(diphenylphosphanyl)pyrrolidine
(*R*,*R*)-Me-DUPHOS = (-)-1,2-Bis[(2*R*,5*R*)-2,5-dimethylphospholanyl]benzene
(*R*,*R*)-Et-DUPHOS = (-)-1,2-Bis[(2*R*,5*R*)-2,5-diethylphospholanyl]benzene
(*R*,*R*)-DIPAMP = (1*R*,2*R*)-Bis[(2-methoxyphenyl)phenylphosphanyl]ethane
(*R*,*R*)-bdpch = (1*R*,2*R*)-(*trans*)-1,2-Bis(diphenylphosphanyloxy)cyclohexane
(*S*,*S*)-DIOP = (4*S*,5*S*)-(+)-4,5-Bis(diphenylphosphanylmyrthyl)-2,2-dimethyl-1,3-dioxolane
Ph-β-Glup = Phenyl 4,6-*O*-benzylidene-2,3-bis(*O*-diphenylphosphanyl)-β-D-glucopyranoside
(*R*)-BINAP = (*R*)-2,2'-Bis(diphenylphosphanyl)-1,1'-binapthyl
(*R*)-Tol-BINAP = (*R*)-2,2'-Bis(di-p-tolylphosphanyl)-1,1'-binapthyl.

For example Ru-BINAP catalysts as described in Tetrahedron Lett. 1991, 32, 4163 and WO 95/18784, the content of these documents is incorporated herein by reference.

In the above ligands the configuration is given as example only. It is understood that the skilled person will choose the configuration of the ligand such that the obtained hydrogenated product has the desired configuration.

The hydrogenation can be carried out under usual conditions, for example in the presence of hydrogen at about room temperature, such as 25°C, under elevated pressure for example in the range of 20 - 80 bar, preferably 30 - 70 bar and in particular 40 - 50 bar. The reaction can be carried out in a suitable solvent, preferably a polar protic solvent, in particular a C₁₋₆ alcohol, such as methanol or ethanol, or an ester, such as ethyl acetate. The reaction can be carried out until hydrogen consumption is finished.

The compound of the formula I prepared by the process of the present invention is intended as intermediate for the preparation of pyrrole derivatives. These derivatives can be prepared by reacting the compound of the formula I with a diketone in a cyclization reaction to obtain the desired pyrrole derivative as described in more detail below. It has now surprisingly been found that if Y in the compound of the formula I is ethyl oligomerization may occur under specific cyclization conditions (elevated temperature, carboxylic acid catalysis). Surprisingly this problem does not occur with the corresponding tert-butyl ester. Therefore, Y in the compound of the formula I is preferably tert-butyl.

However, when trying to synthesize the required tert-butyl ketoester of the formula IV starting from commercially available β-AlaOEtxHCl using traditional Knoevenagel reaction in the last step, while the desired ketoester was formed it was contaminated with about 15% tert-butyl acetoacetate. No reasonable method of purification was found.

While the mono tert-butyl malonate required in the preparation of the intermediate compound of the formula III is commercially available, this compound is expensive and therefore not feasible for use in a commercial process. Therefore, the inventors tried to obtain the required mono tert-butyl malonate by reaction of Meldrum's acid with tert-butanol according to D.W. Brooks, et al., Tedrahedron Lett., 1984, 25, 4623-26 and S. Masayuki, et al., Tetrahedron Lett., 1997, 38, 4623-26 (see reaction scheme 2). It was found that refluxing of Meldrum's acid in excess of tert-butanol gives the desired mono tert-butyl malonate as the main product but also the cyclic compound depicted in scheme 2 as the side product in a ratio of about 30:1.

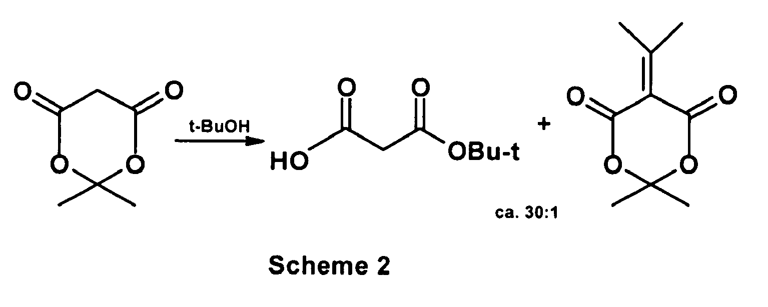

Purification of mono tert-butyl malonate by distillation was unsuccessful since disproportionation of the material to di-tert-butyl malonate and malonic acid was observed.

Surprisingly it was found that a fast purification of mono tert-butyl malonate via its ammonium salt is possible. This salt can be isolated with a yield of 86.7%. Treatment of this salt with hydrochloric acid affords pure (NMR) mono tert-butyl malonate with a yield of 94.3%.

Therefore, the present invention also comprises a process for the preparation of a compound of the formula VI 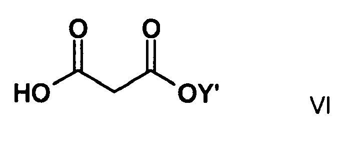 wherein Y' is a carboxy protecting group, which process comprises the steps of reacting Meldrum's acid 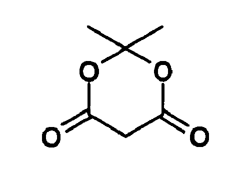 with a compound of the formula Y'OH, wherein Y' is defined as above, and purifying the obtained mono protected malonate via its ammonium salt.

In this process Y' preferably is tert-butyl.

Advantageously this process is combined with the above process for the preparation of a compound of the formula I by converting the compound of the formula IV into its magnesium salt which is then used as intermediate of the formula III.

In the process of the present invention for the preparation of the compound of the formula I reaction step (a) can be carried out under conditions as described for example in T. Honore, et al., Eur. J. Med. Chem. Chim. Ther., 1978, 13, 429-34.

The compounds of the formula I are intended as intermediates for the preparation of pyrrole derivatives for example by cyclization of a diketone. Preferably the process of the present invention therefore comprises the further step of reacting the compound of the formula I, wherein Z is H, with a compound of the formula VII 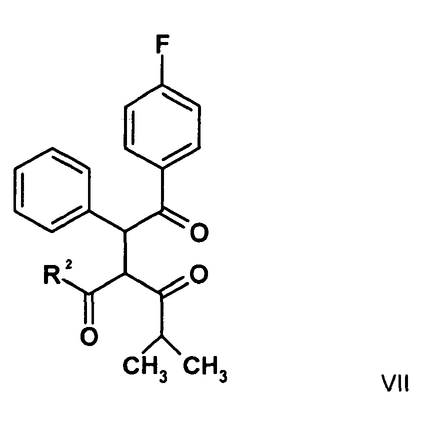 wherein R² is -OR³, -NR⁴R⁵, -NR¹⁰CONR¹¹R¹², -NR¹³OR¹⁴, -ONR¹⁵R¹⁶ or halogen, R³, R⁴, R⁵, R⁶, R⁷, R⁸, R⁹, R¹⁰, R¹¹, R¹², R¹³, R¹⁴ , R¹⁵ and R¹⁶ are independently selected from hydrogen or a straight, branched and/or cyclic, saturated or unsaturated C₁₋₁₀ alkyl residue or aryl residue, both residues being optionally substituted with 1-3 optionally protected hydroxy or carboxy groups, 1-3 -OR⁷ residues, 1-3 -NR⁸R⁹ residues and/or 1-3 halogen atoms, the C₁₋₁₀ alkyl residue optionally comprising 1-3 oxygen atoms, 1-3 nitrogen atoms and/or 1-3 -NR⁶- residues, the C₁₋₁₀ alkyl residue further optionally comprising or being substituted with 1 or 2 aryl residues, to obtain a compound of the formula VIII 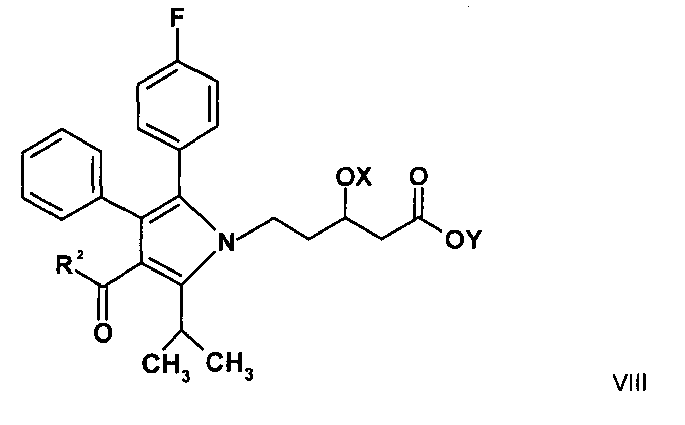 wherein R², X and Y are defined as above.

One advantage of the process of the present invention is that if R² is -OR³ or halogen the compound of the formula VII is easy to produce in good yield and purity. A further advantage is that if R² is -OR³ or halogen the intermediate compound of the formula VII provides the possibility of easy substitution of the R² residue, thereby providing higher flexibility for example for screening substituted pyrrole compounds for their pharmaceutical activity. This finding is particularly unexpected, since the corresponding known diketone anilide (2-[2-(4-fluorophenyl)-2-oxo-1-phenyl-ethyl]-4-methyl-3-oxo-pentanoic acid phenylamide) turned out to be stable to hydrolysis. It reacts with base and hydrogen peroxide not to the expected acid but it breaks to 1-(4-fluorophenyl)-2-phenylethan-1-one.

This problem does not arise when using the corresponding diketone ester or acid halogenide.

For R¹, R³, R⁴, R⁵, R⁶, R⁷, R⁸, R⁹, R¹⁰, R¹¹, R¹², R¹³, R¹⁴, R¹⁵ and R¹⁶ the alkyl residue is preferably a straight or branched, saturated or unsaturated C₁₋₆ alkyl residue or a cyclic C₃₋₆ alkyl residue, such as methyl, ethyl, n-propyl, iso-propyl, n-butyl, iso-butyl, tert-butyl, pentyl, n-hexyl or cyclohexyl. Preferred aryl residues are phenyl and naphthyl, particularly preferred phenyl. The aryl residue furthermore includes heteroaryl residues such as pyrrole or pyridine.

R² preferably is -OR³ or halogen, in particular -OR³.

In the present application halogen stands for fluoro, chloro, bromo or iodo, preferably chloro or bromo.

The reaction between the diketo ester of the formula VII and the amine of the formula I can be carried out in any common inert solvent such as THF, preferably at elevated temperatures, such as, for example, under reflux. Further detailed reaction conditions can for example be found in WO 2004/046105, WO 94/20492 and EP-A-0 330 172. For example, the reaction can be conducted under heating with azeotropical water removed in an appropriate solvent or mixture of solvents catalyzed with an acid, such as, for example, pivalic acid. As suitable solvents n-heptane, toluene and THF or mixtures thereof can be mentioned. Alternatively the reaction can be conducted without any solvent (neat) under heating the reactants to for example about 120°C to 140°C.

The compound of the formula VII can be obtained by
a) reacting a compound of the formula XXIV 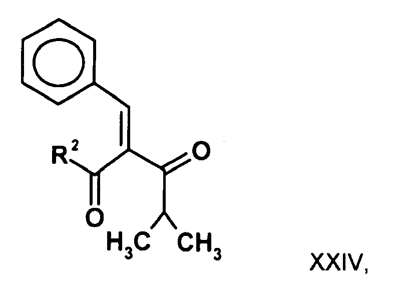 wherein R² is defined as above,
   with 4-fluorobenzaldehyde or
b) reacting a compound of the formula XXV 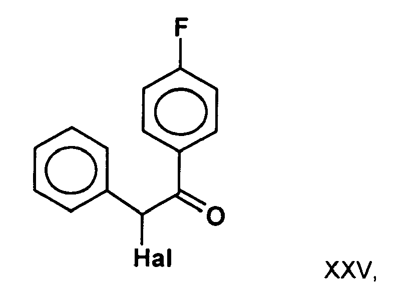 wherein Hal is halogen, preferably bromo with a compound of the formula XXVI 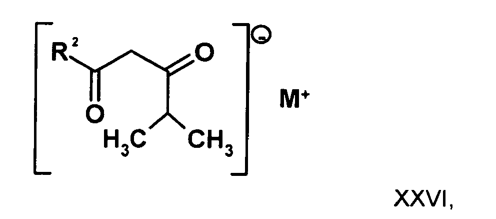 wherein R² is defined as above and M⁺ is selected from H⁺, Li⁺, Na⁺ and K⁺, preferably Na⁺.

The above reaction route a) has the advantage that the compound of the formula XXIV wherein R² is -OR³ can be easily purified by distillation. Thus, this intermediate can be employed in the following reaction in a highly pure form.

The above reaction route b) has the advantage that the compound of the formula XXV can easily be obtained from the commercially available 2-[2-(4-fluorophenyl)-2-oxo-1-phenylethyl]-4-methyl-3-oxo-pentanoic acid phenylamide by cleavage with hydrogen peroxide in the presence of a base such as NaOH.

Above reaction a) can for example be carried out under solvent-free conditions catalyzed by 2-(2-hydroxyethyl)-3-methyl-4-ethylthiazolium bromide in the presence of triethylamine under heating to for example to a temperature of about 60°C to about 80°C.

In reaction route a) the compound of the formula XXIV can be obtained by reacting a compound of the formula XXVII 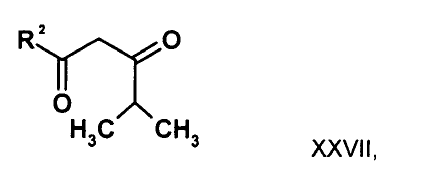 wherein R² is defined as above with benzaldehyde. This reaction can be carried out in the presence of a catalyst such as, for example, piperidine and glacial acetic acid, ethylene diamine and glacial acetic acid, β-alanine and glacial acetic acid, and the like in an inert solvent such as, for example, toluene, heptane, hexane, and the like for about 24 to about 36 hours at about 60°C to about 120°C with the removal of water to afford a compound of the formula XXIV.

If in the compound of the formula XXIV R² is -OR³, this compound is heat-stable and can be distilled as to separate the desired compound of the formula XXIV from undesired byproducts of the reaction. In a preferred embodiment the reaction between the compound of the formula XXVII and benzaldehyde is catalyzed with β-alanine in the presence of acetic acid, preferably glacial acetic acid, in toluene. The reaction mixture is heated under reflux with azeotropic removal of water for about 24 hours. After usual workup the product can be distilled under reduced pressure.

In the alternative embodiment according to above route b) the compound of the formula VII is obtained by reacting the compounds of the formula XXV and XXVI with each other. This addition reaction can be carried out in any suitable inert organic solvent, preferably anhydrous inert organic solvent, such as, for example, ethers, e.g. diethyl ether, 1,2-diethoxyethane, 1,2-dimethoxyethane, THF or mixtures thereof. Preferably the reaction is carried out in THF. The reaction temperature may be in the range of for example about 0°C to about 40°C, preferably from about 0°C to about room temperature.

In a preferred embodiment the compound of the formula XXVI is prepared in situ.

The compound of the formula XXV can be obtained by halogenating the compound of the formula XXVIII

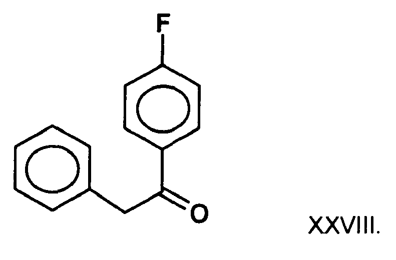

Halogenation, preferably bromination of the compound of the formula XXVIII can be carried out with for example bromine in an inert organic solvent, preferably an anhydrous inert organic solvent, such as, for example, halogenated lower alkane solvents, e.g. CCl₄, CHCl₃, 1,1-dichloroethane, 1,2-dichloroethane, methylene chloride, 1,1,2-trichloroethane or mixtures thereof. A preferred solvent is CHCl₃. The reaction can be carried out for example at a temperature of about 0°C to about 40°C.

The compound of the formula XXVIII can be obtained by cleavage of, for example, the commercially available 2-[2-(4-fluorophenyl)-2-oxo-1-phenyl-ethyl]-4-methyl-3-oxo-pentanoic acid phenylamide. The cleavage can be carried out for example with hydrogen peroxide in the presence of a base such as NaOH.

A particularly advantageous route for the synthesis of a compound of the formula VII is shown in the following scheme 3:

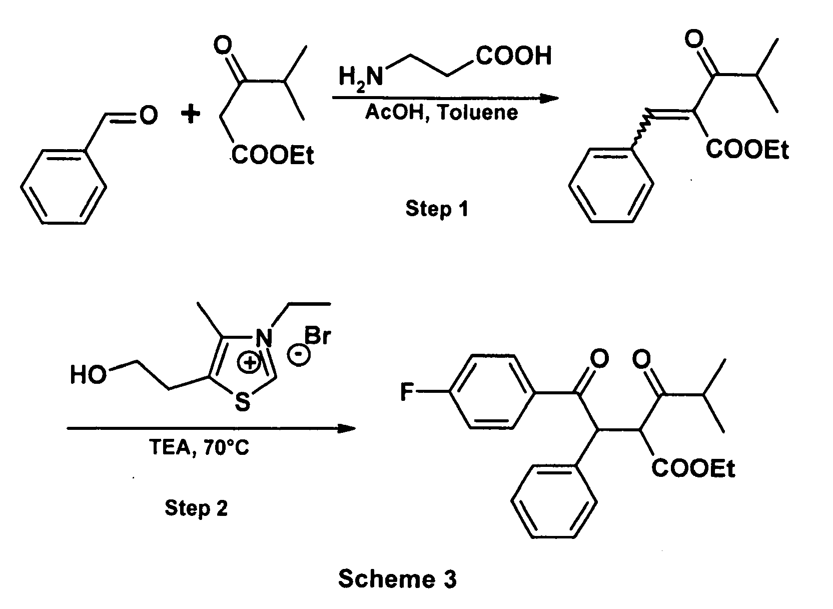

Advantages of the synthesis of diketone ethyl ester by this route are:
- no need of reaction solvent in step 2 (environmental, economic);
- no need of recrystallization process in step 1 (environmental, economic), product is isolated by distillation;
- this is the shortest synthetic route (only 2 steps) in comparison with other known processes (3-5 steps required);
- in the process for the step 1 nontoxic catalyst (β-alanine) is used (environmental);
- no need of toxic a corrosive reagents (like bromine in alternative route);
- all starting materials are commercially available.

The obtained pyrrole derivatives of the formula VIII are useful intermediates in the preparation of atorvastatin. Therefore, the present invention also relates to a process which further comprises the step of converting the compound of the formula VIII into a compound of the formula IX 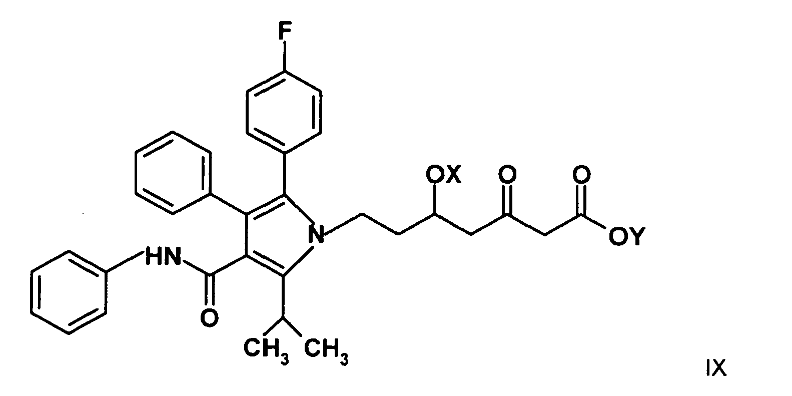 wherein X and Y are defined as above,
by either
a) converting the compound of the formula VIII into a compound of the formula X 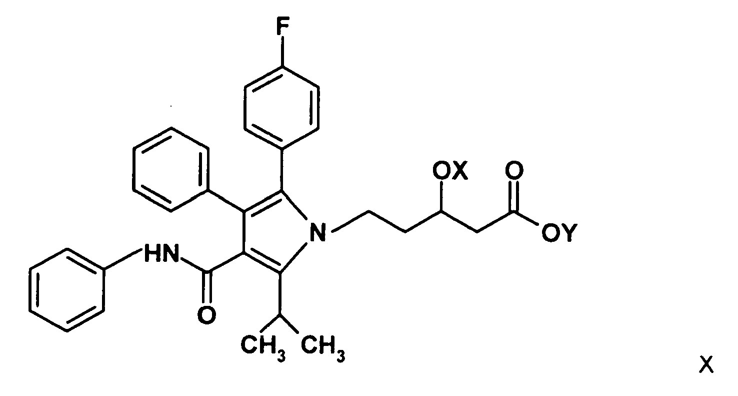 wherein X and Y are defined as above, and subsequently reacting the compound of the formula X with a compound of the formula XI 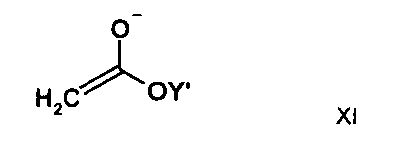 wherein Y' is defined as above, to obtain the compound of formula IX, or
b) reacting the compound of the formula VIII with a compound of the formula XI to obtain a compound of the formula XII 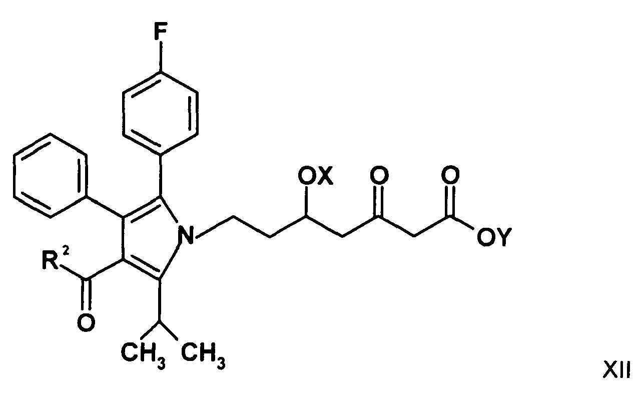 wherein R², X and Y are defined as above, and subsequently converting the compound of the formula XII into a compound of the formula IX.

In this process the compound of the formula XI preferably is tert-butyl acetate.

As the next step the process of the present invention preferably further comprises the step of reducing, in particular hydrating the compound of the formula IX to obtain a compound of the formula XIII 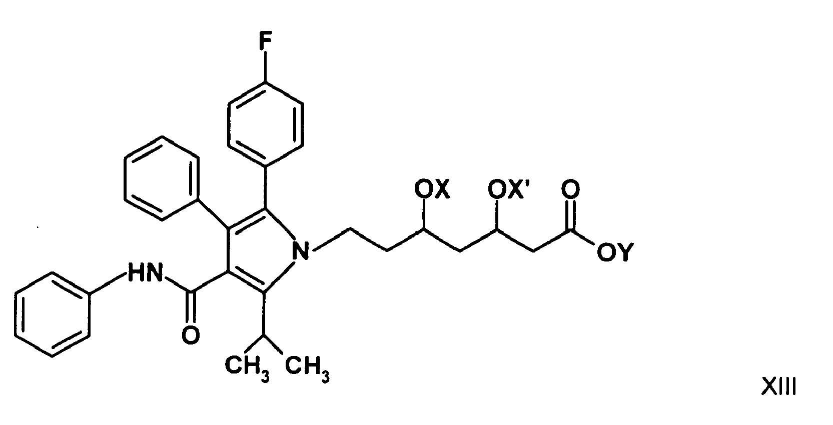 wherein X and Y are defined as above, X' is a hydroxy protecting group or X and X' taken together may form a ring with the oxygen and intermediate carbon atoms to which they are attached, optionally deprotecting any protected groups, and optionally converting the compound of the formula XIII into a salt thereof.

In the process of the present invention the compound of the formula I has preferably the formula I'

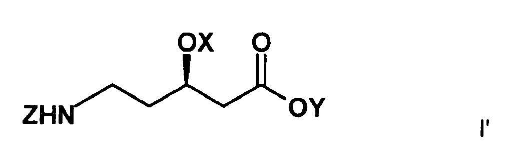

The present invention further relates to compounds of the formula XIV 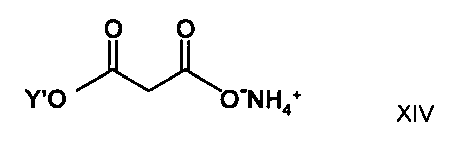 wherein Y' is a carboxy protecting group;
of the formula XV 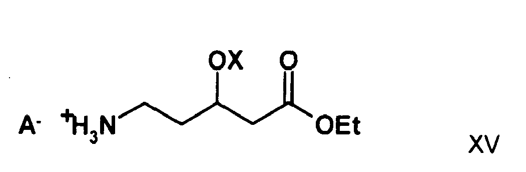 wherein X is H or a hydroxy protecting group and A⁻ is an anion;
of the formula XVI 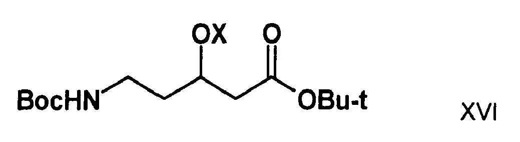 wherein X is H or a hydroxy protecting group; and
of the formula XVII 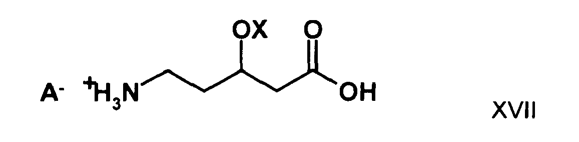 wherein X is H or a hydroxy protecting group and A⁻ is an anion.

These compounds are useful intermediates in the preparation of atorvastatin.

In an alternative embodiment the process of the present invention is a process for the preparation of a pyrrole derivative of the formula XVIII 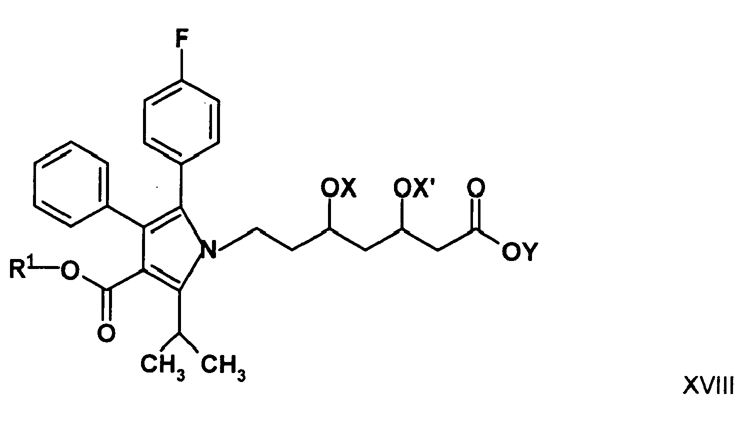 or a salt thereof, wherein R¹ is selected from hydrogen, branched and/or cyclic, saturated or unsaturated C₁₋₁₀ alkyl residue or aryl residue, both residues being optionally substituted with 1-3 optionally protected hydroxy or carboxy groups, 1-3 - OR² residues, 1-3 -NR³R⁴ residues and/or 1-3 halogen atoms, the C₁₋₁₀ alkyl residue optionally comprising 1-3 oxygen atoms, 1-3 nitrogen atoms and/or 1-3 - NR⁵- residues, the C₁₋₁₀ alkyl residue further optionally comprising or being substituted with 1 or 2 aryl residues,
R², R³, R⁴ and R⁵ are independently selected from R¹ as defined above,
X and X' are independently H or a hydroxy protecting group or X and X' taken together may form a ring with the oxygen and intermediate carbon atoms to which they are attached; and
Y is H or a carboxy protecting group; which process comprises the steps of reacting a compound of the formula IXX 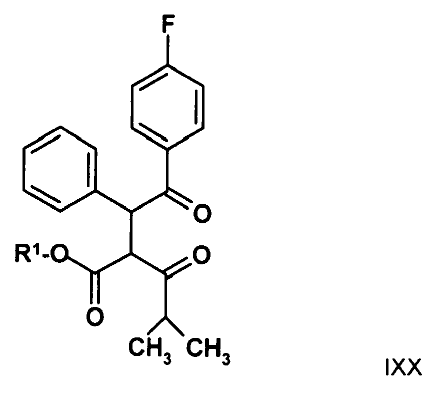 wherein R¹ is defined as above, with a compound of the formula XX 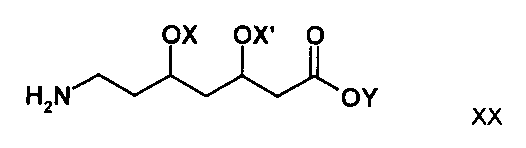 wherein X, X' and Y are defined as above, and optionally removing any of the protecting groups and/or optionally converting the compound of the formula XVIII into a salt thereof.

In this alternative process the compound of the formula XX can be obtained by reacting a compound of the formula XXI 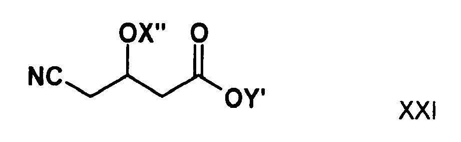 wherein X" is a hydroxy protecting group and Y' is a carboxy protecting group, with a compound of the formula XI 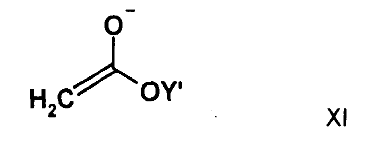 wherein Y' is defined as above, to obtain a compound of the formula XXII 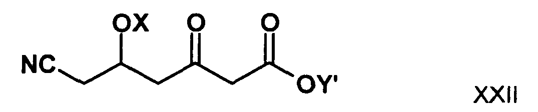 wherein X is H or a hydroxy protecting group and Y' is defined as above, reducing, preferably hydrogenating the compound of formula XXII to obtain a compound of the formula XXIII 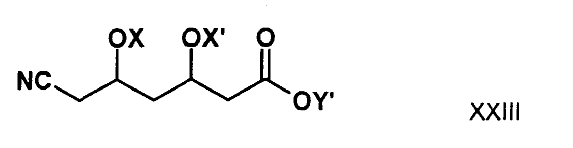 wherein X and X' are independently H or a hydroxy protecting group or X and X' taken together may form a ring with the oxygen and intermediate carbon atoms to which they are attached; and converting the NC- residue in the compound of formula XXIII into a H₂N- residue.

Preferably the compound of formula XXIII is 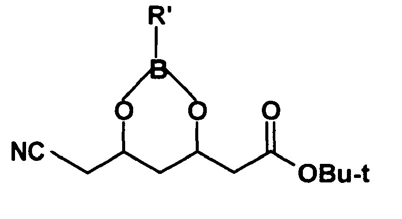 wherein R' is C₁₋₁₀ alkyl or aryl, preferably phenyl.

The obtained pyrrole derivative of the formula XVIII may subsequently be converted into astorvastatin.

The present invention will now be further illustrated by the following examples which are not intended to be limiting.

### Examples

### 1. Ethyl 5-tert.-butoxycarbonylamino-3-oxopentanoate (2)

To a solution of 9.24 g (0.0489 mol) of Boc-β-AlaOH (1) in 110 ml of THF 8.72 g (0.0538 mol) of Im₂CO were added by portions with stirring. The resulted solution was stirred for 2 h at RT after which 15.7 g (0.0548 mol) of Mg(O₂CCH₂CO₂Et)₂ were added. The mixture was stirred at RT overnight. It was concentrated on rotatory evaporator, and the residue was dissolved in AcOEt-water mixture. Water was separated and organic layer was washed with the excess of 2N NaHSO₄ solution (till acidic washings), brine and dried over Na₂SO₄. Evaporation of the solution afforded 12.47 g (98.3 %) of keto ester 2 as yellowish oil which was used in the next stage without additional purification.

¹H NMR, δ (ppm, CDCl₃): 1.28 (t, 3H, J = 7.1 Hz, OCH₂CH₃), 1.43 (s, 9H, Me₃), 2.79 (t, 2H, J = 5.8 Hz, 4-CH₂), 3.32-3.42 (m, 2H, 5-CH₂), 3.46 (s, 2H, 2-CH₂), 4.2 (q, 2H, J = 7.1 Hz, OCH₂CH₃), 5.09 (broad s, 1H, NH).

¹³C NMR, δ (ppm, CDCl₃): 14.26 (OCH₂CH₃), 28.54 (CMe₃), 35.20 (4-CH₂), 43.24 (2-CH₂), 49.52 (5-CH₂), 61.63 (OCH₂CH₃), 79.46 (CMe₃), 156.02 (NCOO), 167.11 (COO), 202.57 (>C=O).

### 2. Ethyl 5-tert.-butoxycarbonylamino-3-hydroxypentanoate (3)

Autoclave (30ml) was charged with 5.2 g (0.020 mol) of keto ester 2 and 7.3 mg (0.0101 mmol) of [Rh(dppb)COD]BF₄ catalyst. The autoclave was deoxygenated by 4 vacuum-Ar cycles. Oxygen-free, dry AcOEt was added followed by H₂ (50 bar initial pressure). During hydrogenation H₂ was added maintaining the pressure in interval 40-50 bar. After 20 h hydrogen consumption finished. The resulted solution was evaporated and dried in vacuum affording 5.24 g (100%) of hydroxy ester 3 as yellowish oil which was used in the next stage without additional purification.

¹H NMR, δ (ppm, CDCl₃): 1.27 (t, 3H, J = 7.1 Hz, OCH₂CH₃), 1.44 (s, 9H, Me₃), 1.51-1.72 (m, 2H, 4-CH₂), 2.41-2.55 (m, 2H, 2-CH₂), 2.99-3.27 (m, 1H, 5-CHₐH_{b}), 3.31-3.49 (m, 1 H, 5-CHₐH_{b}), 3.80 (broad s, 1 H, OH), 4.05-4.15 (m, 1 H, 3-CH), 4.16 (q, 2H, J = 7.1 Hz, OCH₂CH₃), 5.11 (broad s, 1H, NH).

¹³C NMR, δ (ppm, CDCl₃): 14.39 (OCH₂CH₃), 28. 63 (CMe₃), 36.85 (CH₂), 37.53 (CH₂), 41.73 (5-CH₂), 60.91 (OCH₂CH₃), 66.18 (3-CH), 79.59 (CMe₃), 156.94 (NCOO), 172.80 (COO).

### 3. Ethyl 5-amino-3-hydroxypentanoate, tosylate (4)

A solution of 5 g (0.0216 mol) of above compound 3 and 5 g (0.0263 mol) of p-TsOHxH₂O in 20 ml of EtOH was kept at 70°C for 1 h. The solvent was evaporated. The residue was washed with Et₂O and dried in vacuum to give 6.75 g (93.7%) of amino ester salt 4 as yellowish oil.

¹H NMR, δ (ppm, CDCl₃): 1.17 (t, 3H, J = 7.1 Hz, OCH₂CH₃), 1.59-1.71 (m, 2H, 4-CH₂), 2.17-2.42 (m, 2H, 2-CH₂), 2.31 (s, 3H, Me), 2.94-3.17 (m, 2H, 5-CH₂), 4.01-4.14 8m, 1H, 3-CH), 4.04 (q, 2H, J = 7.1 Hz, OCH₂CH₃), 7.09 (d, 2H, J = 8 Hz, ArH), 7.25 (broad s, 1 H, OH), 7.37 (broad s, 2H, NH₃), 7.70 (d, 2H, J = 8 Hz, ArH).

¹³C NMR, δ (ppm, CDCl₃): 14.34 (OCH₂CH₃), 21.59 (Me), 32.94 (CH₂), 38.46 (CH₂), 41.89 (5-CH₂), 60.93 (OCH₂CH₃), 66.98 (3-CH), 126.34 (CH), 129.40 (CH), 140.53 (C), 141.41 (C), 172.15 (COO).

### 4. tert.-Butyl 5-tert.-butoxycarbonylamino-3-oxopentanoate (5)

To a solution of 5.52 g (0.0292 mol) of Boc-β-AlaOH (1) in 60 ml of THF 5.21 g (0.0321 mol) of Im₂CO were added by portions with stirring. The resulted solution was stirred for 2 h at RT after which 11.2 g (0.0327 mol) of Mg(O₂CCH₂CO₂Bu-*t*)₂ were added. The mixture was stirred at RT overnight. It was concentrated on rotatory evaporator, and the residue was dissolved in AcOEt-water mixture. Water was separated and organic layer was washed with the excess of 2N NaHSO₄ solution (till acidic washings), brine and dried over Na₂SO₄. Evaporation of the solution afforded 7.80 g (93.0 %) of keto ester 5 as yellowish oil which was used in the next stage without additional purification.

¹H NMR, δ (ppm, CDCl₃): 1.47 (t, 9H, Me₃), 1.43 (s, 9H, Me₃), 2.77 (t, 2H, J = 5.6 Hz 4-CH₂), 3.34-3.42 (m, 2H, 5-CH₂), 3.38 (s, 2H, 2-CH₂), 5.12 (broad s, 1H, NH).
¹³C NMR, δ (ppm, CDCl₃): 28.16 (CMe₃), 28.57 (CMe₃), 35.21 (4-CH₂), 43.14 (2-CH₂), 50.84 (5-CH₂), 82.43 (CMe₃), 82.73 (CMe₃), 166.49 (COO), 203.22 (>C=O).

### 5. tert.-Butyl 5-tert.-butoxycarbonylamino-3-hydroxypentanoate (6)

Autoclave (30 ml) was charged with 2.9 g (0.0101 mol) of keto ester 5 and 7.3 mg (0.0101 mmol) of [Rh(dppb)COD]BF₄ catalyst. The autoclave was deoxygenated by 4 vacuum-Ar cycles. Oxygen-free, dry AcOEt was added followed by H₂ (50 bar initial pressure). During hyrogenation H₂ was added maintaining the pressure in interval 40-50 bar. After 24 h hydrogen consumption finished. The resulted solution was evaporated and dried in vacuum affording 2.9 g (100 %) of hydroxy ester 6 as yellowish oil which was used in the next stage without additional purification.

¹H NMR, δ (ppm, CDCl₃): 1.44 (s, 9H, Me₃), 1.46 (s, 9H, Me₃), 1.48-1.72 (m, 2H, 4-CH₂), 2.36-2.43 (m, 2H, 2-CH₂), 3.11-3.26 (m, 1H, 5-CHₐH_{b}), 3.30-3.47 (m, 1 H, 5-CHₐH_{b}), 3.72 (broad s, 1H, OH), 4.00-4.10 (m, 1H, 3-CH), 5.07 (broad s, 1H, NH).
¹³C NMR, δ (ppm, CDCl₃): 28.37 (CMe₃), 28.69 (CMe₃), 36.68 (CH₂), 37.71 (CH₂), 42.69 (5-CH₂), 66.54 (3-CH), 79.54 (CMe₃), 81.47 (CMe₃), 156.83 (NCOO), 172.37 (COO).

### 6. 5-Amino-3-hydroxypentanoic acid, tosylate (7)

A solution of 0.7 g (0.00242 mol) of above compound 6 and 0.56 g (0.00294 mol) of p-TsOHxH₂O in 20 ml of EtOH was kept at 70°C for 1 h. The solvent was evaporated. The residue was washed with Et₂O and dried in vacuum to give 0.87 g (99.5 %) of amino ester salt 7 as yellowish oil.

¹H NMR, δ (ppm, CD₃OD): 1.7-1.96 (m, 2H, 4-CH₂), 2.40 (s, 3H, Me), 2.48-2.56 (m, 2H, 2-CH₂), 3.03-3.17 (m, 2H, 5-CH₂), 4.09-4.20 (m, 1 H, 3-CH), 7.28 (d, 2H, J = 8 Hz, ArH), 7.75 (d, 2H, J = 8 Hz, ArH).
¹³C NMR, δ (ppm, CDCl₃): 21.17 (Me), 35.46 (CH₂), 39.37 (CH₂), 43.78 (5-CH₂), 68.24 (3-CH), 127.75 (CH), 130.73 (CH), 142.72 (C), 144.09 (C), 174.15 (COO).

### 7. Ethyl-2-benzylidene-4-methyl-3-oxopentanoate (compound of formula XXIV)

A mixture of benzaldehyde (33.8 g, 319 mmol), ethyl isobutyrylacetate (50.4 g, 319 mmol), β-alanine (1.0 g) and acetic acid (10 ml) in 600 ml of toluene was stirred and heated under reflux with azeotropic removal of water (Dean-Stark adapter) until GC analysis showed no presence of starting material (24 h). The solution was cooled, poured into ethylacetate (400 ml), washed with 1 M HCl solution (2 x 100 ml), saturated NaHCO₃ solution (2 x 100 ml) and brine (100 ml) and dried over anhydrous Na₂SO₄. The solvent was removed under reduced pressure to yield 76.3 g of brown oil which was further distilled under reduced pressure (bp 105 - 120°C/0.06 mm Hg) to afford 58.9 g (75%) of ethyl 2-benzylidene-4-methyl-3-oxopentanoate as a mixture of isomers (major ~ 70%).

### 8. Ethyl 2-[2-(4-fluorophenyl)-2-oxo-1-phenyl-ethyl]-4-methyl-3-oxopentanoate (compound of formula VII)

To a stirred mixture of ethyl 2-benzylidene-4-methyl-3-oxopentanoate (52.5 g, 0.213 mol), 4-fluorobenzaldehyde (39.7 g, 0.320 mol) and 2-(2-hydroxyethyl)-3-methyl-4-ethylthiazolium bromide (8.11 g, 0.032 mol) was added dropwise triethylamine (21.3 ml). The solution turned dark and precipitation of solid particles was observed. A mixture was then heated at 70°C until the reaction mixture did not contain starting ethyl 2-benzylidene-4-methyl-3-oxopentanoate (HPLC monitoring). After cooling to room temperature, the mixture was diluted with ethylacetate (500 ml) and water (100 ml). The organic phase was washed with 1 M HCl solution (2 x 100 ml), saturated NaHCO₃ solution (2 x 100 ml) and brine (100 ml) and dried over anhydrous Na₂SO₄. The solvent was removed under reduced pressure to yield 100.2 g of impure oily product which was then dissolved in dichloromethane (400 ml) and silicagel (50 g) was added. The mixture was stirred for 10 minutes and the silicagel was filtered off and washed with dichloromethane (500 ml). Air was bubbled through the combined filtrates for 24 hours in order to oxidize remaining 4-fluorobenzaldehyde. Additional 200 ml of dichloromethane was added to the reaction mixture to compensate losses due to evaporation and the solution was washed with saturated NaHCO₃ solution (2 x 100 ml) and water (100 ml) and dried over anhydrous Na₂SO₄. The solvent was removed under reduced pressure to yield 87 g yellow oily product which slowly solidified. The oily product contains 85% of ethyl 2-[2-(4-fluorophenyl)-2-oxo-1-phenyl-ethyl]-4-methyl-3-oxopentanoate and the calculated yield was then 94%.

### 9. O-tert.-Butylmalonic acid ammonium salt (7)

A mixture of 50 g (0.347 mol) of Meldrum's acid and 100 ml of *t*-BuOH (Merck, for synthesis quality) was gently refluxed for 5 h with CaCl₂ tube on the head of the reflux condenser. After cooling excess of *t*-BuOH and formed acetone were removed on rotatory evaporator. The residue was dissolved in ca. 200 ml of absolute EtOH, the solution was cooled in ice bath, and 26 ml of 25% ammonia solution (0.348 mol) were added. The volatiles were evaporated on rotor evaporater until solid residue was formed. This was triturated under 200 ml of ether, filtered and washed with the same volume of ether. Drying on air afforded 53.9 g (87.6%) of ammonium salt 7, m.p. 93-97°C. In CD₃OD H-D exchange in CH2 group is observed. ¹H NMR (CD₃OD), δ (ppm): 1.49 (*t*-Bu); 3.09-3.31 (residual CH₂ and CHD), 5.19 (H₂O, DOH). ¹³C NMR (CD₃OD), δ (ppm): 29.19 (Me), 47-48 (m, residual CHD), 82.6 (Me₃C), 171.5 and 175.3 (COO). Anal. Calcd for C₇H₁₅NO₄: C, 47.45; H, 8.53; N, 7.90. Found: C, 47.01; H, 8.50; N, 8.03.

### 10. tert.-Butyl malonate (8)

A mixture of 50 g of ammonium salt 7 and 300 ml of ether was cooled in ice bath, and 100 ml of ca. 9% HCl solution was slowly added. After 30 min organic layer was separated and washed with brine (3x100 ml). Ether solution was dried over Na₂SO₄ and evaporated. The residue was dried in high vacuum till constant pressure. Yield 42.6 g (94.3%). ¹H NMR (CDCl₃), δ (ppm): 1.49 (s, 9H, Me₃), 3.36 (s, 2H, CH₂) 11.59 (broad s, 1H, COOH). ¹³C NMR (CDCl₃), δ (ppm): 28.11 (Me), 42.32 (CH₂), 83.12 (Me₃C), 166.43 and 172.47 (COO). Anal. Calcd for C₇H₁₂O₄: C, 52.49; H, 7.55. Found: C, 51.81; H, 7.35.

## Claims

1. Process for the preparation of a compound of the formula I 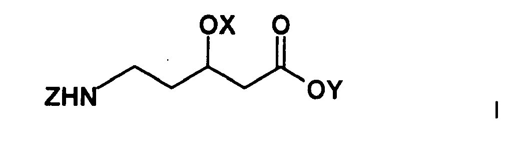 or a salt thereof, wherein Z is H or an amino protecting group, X is H or an hydroxy protection group, and Y is H or a carboxy protecting group, which process comprises the steps of
a) reacting a compound of the formula II 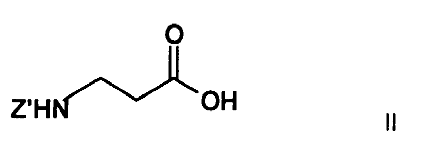 wherein Z' is an amino protecting group, with N,N'-carbonyldiimidazole (Im₂CO) and a compound of the formula III 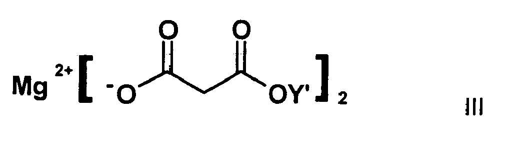 wherein Y' is a carboxy protecting group, to a compound of the formula IV 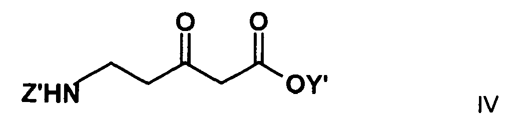 wherein Z' and Y' are defined as above,
b) reducing the compound of the formula IV to obtain a compound of the formula V 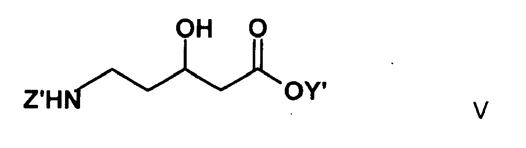 wherein Z' and Y' are defined as above, and
c) optionally deprotecting the amino group and/or the carboxy group of the compound of the formula V, optionally protecting the hydroxy group of the compound of the formula V and/or optionally converting the resulting compound into a salt thereof.

2. Process according to claim 1, wherein Z' is tert-butoxycarbonyl (Boc) and Y' is alkyl, aryl or aralkyl, preferably methyl, ethyl, iso-propyl or tert-butyl.

3. Process according to any of the preceding claims, wherein the reduction step (b) is a hydrogenation which preferably is carried out in the presence of a chiral catalyst.

4. Process according to claim 3, wherein the chiral catalyst is selected from chiral iridium, rhodium and ruthenium complexes.

5. Process for the preparation of a compound of the formula VI 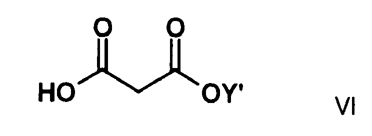 wherein Y' is a carboxy protecting group, which process comprises the steps of reacting Meldrum's acid 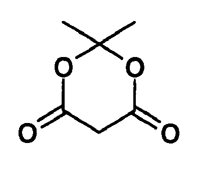 with a compound of the formula Y'OH, wherein Y' is defined as above, and purifying the obtained mono protected malonate via its ammonium salt.

6. Process according to any of claims 1-4, wherein the compound of the formula III is obtained by a process comprising the steps of claim 5 and converting the compound of the formula VI into the magnesium salt thereof.

7. Process for preparing a compound of the formula I 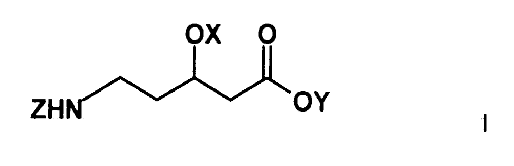 wherein Z is H or an amino protecting group, X is H or a hydroxy protecting group and Y is H or a carboxy protecting group, which process comprises the step of homogenous achiral or chiral enantioselective hydrogenation of a compound of the formula IV 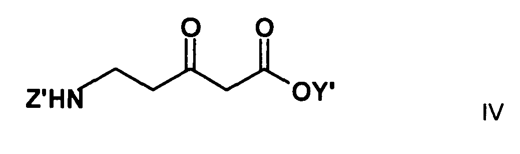 wherein Z' is an amino protecting group and Y' is a carboxy protecting group, and optionally deprotecting and/or protecting any protected or unprotected group.

8. Process according to any of claims 1-4 or 6, which comprises the steps of claim 7.

9. Process according to any of claims 1-4, 6 or 8, further comprising the step of reacting the compound of the formula I, wherein Z is H, with a compound of the formula VII 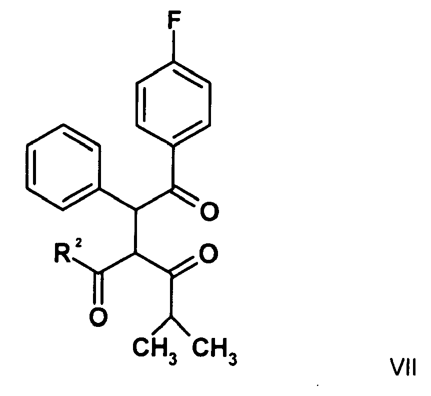 wherein R² is -OR³, -NR⁴R⁵, -NR¹⁰CONR¹¹R¹², -NR¹³OR¹⁴, -ONR¹⁵R¹⁶ or halogen,
R³, R⁴, R⁵, R⁶, R⁷, R⁸, R⁹, R¹⁰, R¹¹, R¹², R¹³, R¹⁴, R¹⁵ and R¹⁶ are independently selected from hydrogen or a straight, branched and/or cyclic, saturated or unsaturated C₁₋₁₀ alkyl residue or aryl residue, both residues being optionally substituted with 1-3 optionally protected hydroxy or carboxy groups, 1-3 -OR⁷ residues, 1-3 -NR⁸R⁹ residues and/or 1-3 halogen atoms, the C₁₋₁₀ alkyl residue optionally comprising 1-3 oxygen atoms, 1-3 nitrogen atoms and/or 1-3 -NR⁶- residues, the C₁₋₁₀ alkyl residue further optionally comprising or being substituted with 1 or 2 aryl residues, to obtain a compound of the formula VIII 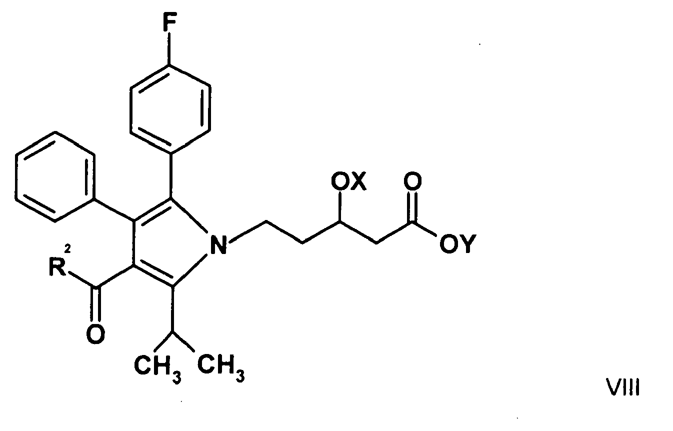 wherein R² is defined as above and X and Y are defined as in claim 1.

10. Process according to claim 9 further comprising the step of converting the compound of the formula VIII into a compound of the formula IX 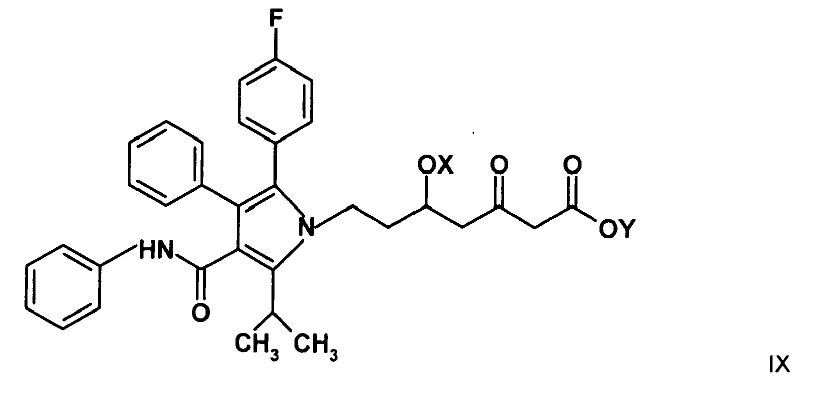 wherein X and Y are defined as in claim 1,
by either
a) converting the compound of the formula VIII into a compound of the formula X 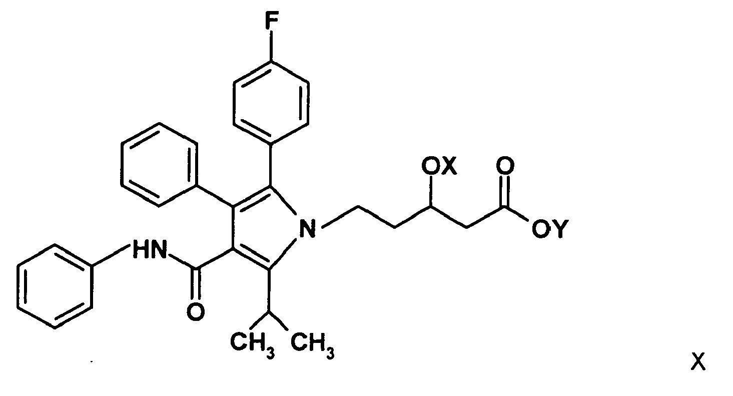 wherein X and Y are defined as above, and subsequently reacting the compound of the formula X with a compound of the formula XI 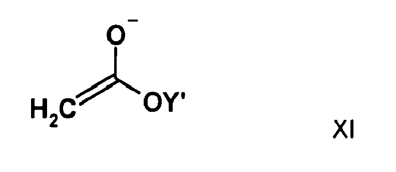 wherein Y' is defined as in claim 1, to obtain the compound of formula IX, or
b) reacting the compound of the formula VIII with a compound of the formula XI to obtain a compound of the formula XII 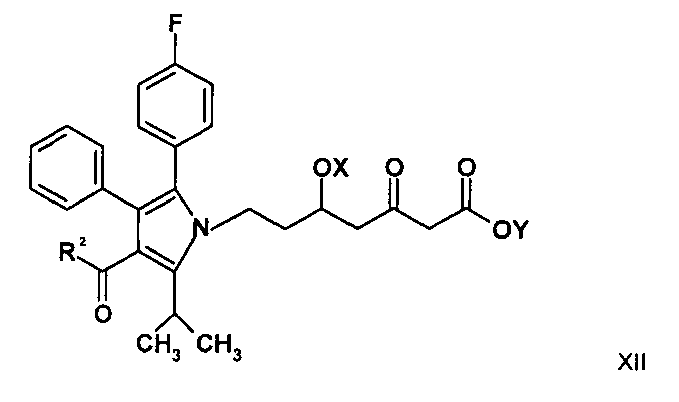 wherein R² is defined as in claim 9 and X and Y are defined as in claim 1, and subsequently converting the compound of the formula XII into a compound of the formula IX.

11. Process of claim 10, wherein the compound of the formula XI is tert-butyl acetate.

12. Process of claim 10 or 11, further comprising the step of reducing the compound of the formula IX to obtain a compound of the formula XIII 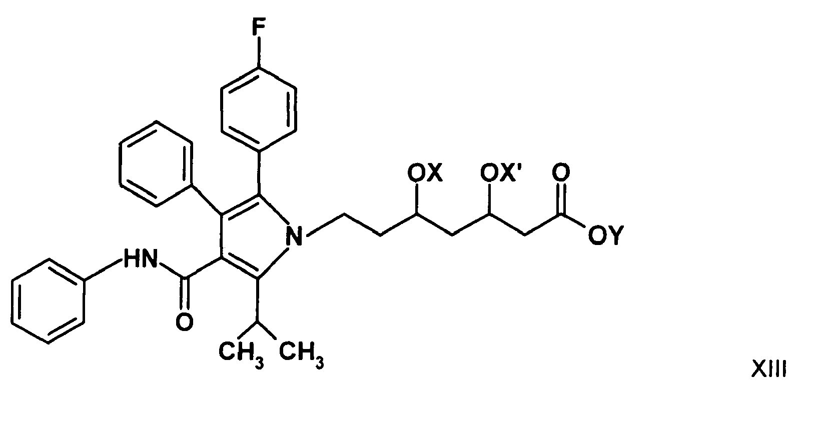 wherein X and Y are defined as in claim 1, X' is a hydroxy protecting group or X and X' taken together may form a ring with the oxygen and intermediate carbon atoms to which they are attached, optionally deprotecting any protected groups, and optionally converting the compound of the formula XIII into a salt thereof.

13. Process according to any of claims 1-4, 6 and 8-12, wherein the compound of the formula I has the formula I' 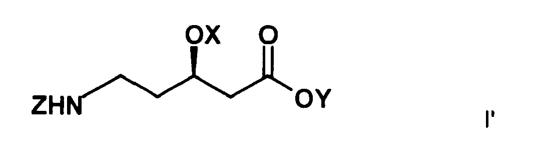 wherein Z, X and Y are defined as in claim 1.

14. Compound of the formula XIV 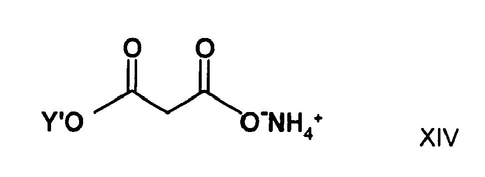 wherein Y' is a carboxy protecting group.

15. Compound of the formula XV 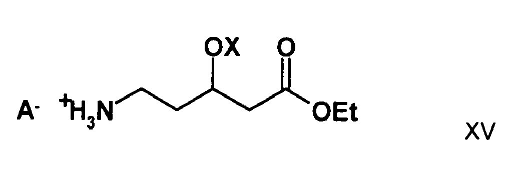 wherein X is H or a hydroxy protecting group and A⁻ is an anion.

16. Compound of the formula XVI 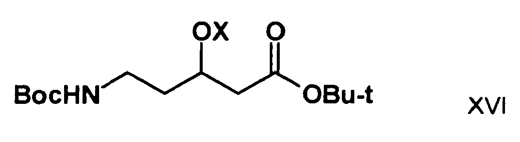 wherein X is H or a hydroxy protecting group.

17. Compound of the formula XVII 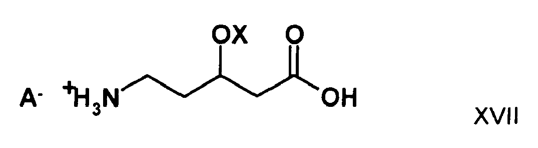 wherein X is H or a hydroxy protecting group and A⁻ is an anion.

18. Use of a compound of any of claims 14-17 for the preparation of atorvastatin.

19. Process for the preparation of a pyrrole derivative of the formula XVIII 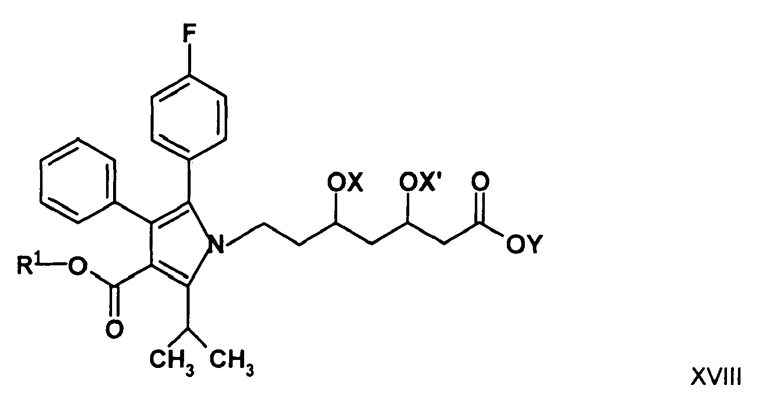 or a salt thereof, wherein R¹ is selected from hydrogen, branched and/or cyclic, saturated or unsaturated C₁₋₁₀ alkyl residue or aryl residue, both residues being optionally substituted with 1-3 optionally protected hydroxy or carboxy groups, 1-3 - OR² residues, 1-3 -NR³R⁴ residues and/or 1-3 halogen atoms, the C₁₋₁₀ alkyl residue optionally comprising 1-3 oxygen atoms, 1-3 nitrogen atoms and/or 1-3 - NR⁵- residues, the C₁₋₁₀ alkyl residue further optionally comprising or being substituted with 1 or 2 aryl residues,
R², R³, R⁴ and R⁵ are independently selected from R¹ as defined above,
X and X' are independently H or a hydroxy protecting group or X and X' taken together may form a ring with the oxygen and intermediate carbon atoms to which they are attached; and
Y is H or a carboxy protecting group; which process comprises the steps of reacting a compound of the formula IXX 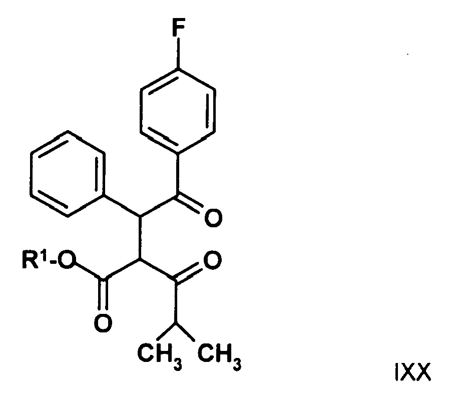 wherein R¹ is defined as above, with a compound of the formula XX 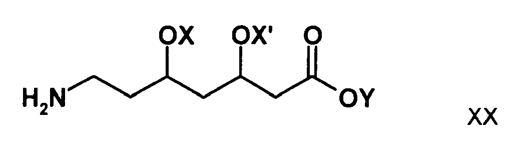 wherein X, X' and Y are defined as above, and optionally removing any of the protecting groups and/or optionally converting the compound of the formula XVIII into a salt thereof.

20. Process according to claim 19, wherein the compound of the formula XX is obtained by reacting a compound of the formula XXI 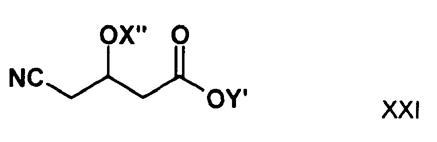 wherein X" is a hydroxy protecting group and Y' is a carboxy protecting group, with a compound of the formula XI 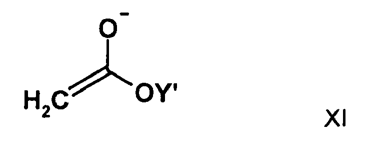 wherein Y' is defined as above, to obtain a compound of the formula XXII 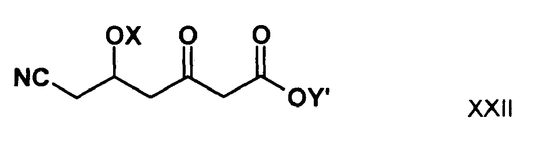 wherein X is H or a hydroxy protecting group and Y' is defined as above, reducing, preferably hydrogenating the compound of formula XXII to obtain a compound of the formula XXIII 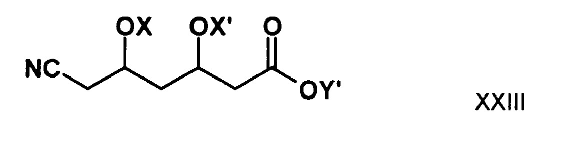 wherein X and X' are independently H or a hydroxy protecting group or X and X' taken together may form a ring with the oxygen and intermediate carbon atoms to which they are attached; and converting the NC- residue in the compound of formula XXIII into a H₂N- residue.

21. Process according to claim 20, wherein the compound of formula XXIII is 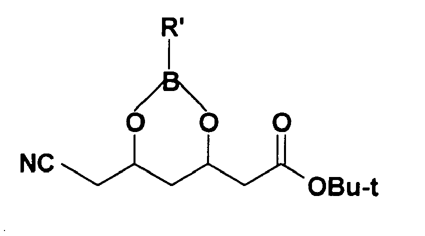 wherein R' is C₁₋₁₀ alkyl or aryl, preferably phenyl.

22. Process according to any of claims 19-21, wherein the pyrrol derivative of formula XVIII is converted into atorvastatin.
